# EUROPÄISCHE PATENTANMELDUNG

(11) **EP 3 404 090 A1**
(43) Veröffentlichungstag der Anmeldung: **21.11.2018**
(21) Anmeldenummer: 17171182.3
(22) Anmeldetag: 15.05.2017
(51) Int. Cl.: C12M 1/00, C12M 1/34, C12M 1/36

(54) **INKUBATOR, SYSTEM UND VERFAHREN FÜR DAS ÜBERWACHTE ZELLWACHSTUM**

(71) Anmelder: Eppendorf AG, 22339 Hamburg (DE)
(72) Erfinder: WENTE, Wolf, 22339 Hamburg (DE); JOLIE, Christoph, 22339 Hamburg (DE)
(74) Vertreter: Kirchner, Christian

(57) **Zusammenfassung**

Die Erfindung bezieht sich auf einen Inkubator, ein System und ein Verfahren zur Überwachung des Zellwachstums einer Zellkultur durch Erfassung von Zellüberwachungsdaten im Inkubator. Der Inkubator weist eine Datenspeichereinrichtung (4a), und eine Datenverarbeitungseinrichtung (4b) auf, die zum Erfassen mindestens eines Inkubatorkammerwertes und mindestens eines Zellwachstumswertes in Form von Zellüberwachungsdaten und zum Speichern der Zellüberwachungsdaten in der Datenspeichereinrichtung eingerichtet ist.

## Beschreibung

Die Erfindung betrifft einen Inkubator für das überwachte Wachstum von biologischen Zellen. Die Erfindung betrifft zudem ein System und ein Verfahren für das überwachte Wachstum von biologischen Zellen.

Mit solchen Inkubatoren werden in biologischen und medizinischen Laboratorien Zellen in Zellkultur unter kontrollierten Umgebungsbedingungen gehalten, und so das Wachstum lebender Zellen *in vitro* ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen benötigen CO₂-Inkubatoren. Die Atmosphäre wird durch Luft mit einem bestimmten CO₂- und O₂-Gehalt und einer bestimmten Luftfeuchtigkeit gebildet, eine geeignete Temperatur ist oftmals 37 °C.

Zellen werden für die verschiedensten Anwendungen in Biologie, Medizin und Pharmazie benötigt. Wichtig ist dabei, dass die Zellen in der gewünschten Beschaffenheit, also in reproduzierbarer Weise bereitgestellt werden. Immortalisierte Zelllinien bieten dazu besonders geeignete Voraussetzungen, da bei diesen ein genetisch einheitlicher Zelltyp und eine nahezu unbegrenzte Anzahl von Zellteilungszyklen möglich ist, sowie die Möglichkeit des Einfrierens (und Auftauens) in bei -196°C temperierten Langzeitdepots.

Das Wachstum der Zellen hängt insbesondere kritisch von den Umgebungsbedingungen im Inkubator ab. Die Inkubatorkammern werden deshalb einerseits regelmäßig durch Reinigungs- und Desinfektionsmaßnahmen behandelt. Andererseits weisen Inkubatoren eine besonders hochwertige Elektronik und Sensorik auf. Die Sensoren werden üblicher Weise regelmäßig kalibriert. In der Praxis kommt es jedoch immer wieder vor, dass trotz vermeintlich akkuratem Befolgen der notwendigen Wartungsmaßnahmen das Wachstum der Zellen nicht die erwarteten Ergebnisse zeigt. Dies gilt selbst für immortalisierte Zelllinien.

Um Informationen über den Wachstumsprozess zu erhalten, werden Zellkulturen bzw. deren Zellen oftmals einer visuellen Inspektion und morphologischen Überprüfung sowie einer Zellzählung außerhalb des Inkubators unterzogen. Diese Maßnahmen führen zu Momentaufnahmen, die einer Beurteilung des Zellwachstums bis zu diesem Zeitpunkt dienen können. Solche Maßnahmen unterbrechen aber das Wachstum der Zellen und stören dieses durch das Bewegen des Zellmediums und durch die zumindest zeitweilige Exposition in der Laborraumatmosphäre. Die zuverlässige Weiterentwicklung der so geprüften Zellen ist fragwürdig. Zudem führt das Entnehmen eines Zellkulturbehälters aus dem Inkubator aufgrund der Störung der Inkubatoratmosphäre beim Öffnen der Inkubator-Türe auch zur Störung der anderen Zellkulturen im Inkubator.

Es sind Zubehörgeräte für die Beobachtung von Zellkulturen bekannt, die in Inkubatoren platziert werden und mit denen auf einem externen Computer bestimmte Informationen über das Wachstum einer Zellkultur gewonnen werden können, ohne dass diese der Inkubatorkammer entnommen werden müssen. Auf diese Weise kann ein Benutzer vom Computer beispielsweise durch Bildübermittlung und/oder Bildauswertung über den Status eines Zellkulturwachstums informiert werden. Weniger zufriedenstellend erscheint bei solchen Lösungen der insgesamt hohe Betriebsaufwand solcher Systeme, betreffend insbesondere die Anschaffung des Zubehörs und des Inkubators, deren Wartung und sowie der Aufwand bei der Bedienung. Zubehörgeräte werden zudem unabhängig vom Inkubator betrieben, so dass durch eine unbedachte Einstellung des Inkubators oder des Zuberhörs durch den Benutzer eine Fehlfunktion oder Beschädigung der Geräte herbeigeführt werden können. Es können z.B. versehentlich temperaturempfindliche Geräte bei einer Hochtemperaturdesinfektion des Inkubators in der Inkubatorkammer verbleiben, bzw. temperaturunempfindliche Geräte unnötiger Weise entnommen werden. Es können auch zeitweilig ungenutzte Zubehörgeräte in der Inkubatorkammer eingeschaltet bleiben und das Klima stören.

Der vorliegenden Erfindung liegt deshalb die Aufgabe zugrunde, Zellen der gewünschten Beschaffenheit in besser reproduzierbarer und effizienter Weise bereitzustellen.

Die Erfindung löst diese Aufgabe durch den Inkubator gemäß Anspruch 1, das Datenaustauschsystem gemäß Anspruch 10, das System gemäß Anspruch 11 und das Verfahren gemäß Anspruch 12. Bevorzugte Ausgestaltungen sind insbesondere Gegenstände der Unteransprüche.
Der erfindungsgemäße Inkubator für das überwachte Wachstum von Zellkulturen, weist auf: eine Inkubatorkammer zur Aufnahme mindestens eines Zellkulturbehälters, der eine darin wachsende Zellkultur enthält, eine Sensoreinrichtung zur Messung mindestens eines Kammerparameters, der einen physikalischen Zustand der Inkubatorkammer charakterisiert, in Form mindestens eines ersten Messwertes, mindestens eine Messeinrichtung zur Messung mindestens eines Wachstumsparameters, der das Wachstum der Zellen dieser Zellkultur charakterisiert, in Form mindestens eines zweiten Messwertes, eine Datenspeichereinrichtung, und eine Datenverarbeitungseinrichtung, die zum Erfassen des mindestens einen ersten Messwertes und des mindestens einen zweiten Messwertes in Form von Zellüberwachungsdaten und zum Speichern der Zellüberwachungsdaten in der Datenspeichereinrichtung eingerichtet ist.

Im Gegensatz zu bekannten Inkubatoren wird mit dem vorliegenden Inkubator eine neue als Informationszentrale dienende Apparategattung eingeführt, die Informationen über das Wachstum der Zellen in dem individuellen Inkubator sammelt und auf diese Weise Referenzdaten gewinnt, die eine standardisierte Vorgehensweise für die Zellkultur in dem Inkubator erlauben. Die hochwertige und präzise kalibrierte Sensorik der Inkubatoren bietet die Grundlage zur Erhebung eines verlässlichen Datensatzes zur Charakterisierung des Zellwachstums in diesem Inkubator unter dessen atmosphärischen Bedingungen, insbesondere CO2, O2, relative Luftfeuchtigkeit, und/oder Anzahl bzw. Dauer der Türöffnungen. Es werden dabei die inkubator-internen Parameter, insbesondere Kammerparameter, gemeinsam mit den durch die Messeinrichtung gemessenen Wachstumsparametern gesammelt, so dass eine Ermittlung von eventuellen Korrelationen zwischen Parameterwerten und deren Änderungen ermöglicht wird. Auf diese Weise werden im Inkubator Zellüberwachungsdaten gewonnen, die als Referenzdaten zur Einschätzung der Entwicklung zukünftig im Inkubator zu wachsender Zellkulturen einsetzbar sind. Durch die gleichzeitige Erfassung der Umweltbedingungen des Inkubators in Form eines ersten Messwertes sowie der Wachstumsparameter in Form eines zweiten Messwertes und auch der Speicherung beider Messwerte liegen diese Informationen in einem einzigen Gerät zentral vor. In der Funktion des Inkubators als Informationszentrale kann dieser Inkubator auch an ein Netzwerk angeschlossen sein und über dieses Netzwerk die erfassten Daten an eine Datenbank, die entweder öffentlich zugänglich oder nur für eine definierten Nutzerkreis zugänglich ist, senden und dort hinterlegen. Durch die Bereitstellung dieser Daten wird es erstmals möglich nutzerübergreifend Informationen über das Wachstum von Zellen unter erfassten Umweltbedingungen zu sammeln, Bereitzustellen und auch zu Dokumentieren. Es ist möglich diese Zellüberwachungsdaten mit den erfassten Umgebungsbedingungsdaten des Inkubators zu verknüpfen. Eine Standardisierung der Zellkultivierung wird so möglich gemacht. Auch wird es erheblich erleichtert die erfassten Zell- und Umgebungsbedingungsdaten von Arbeitsgruppe zu Arbeitsgruppe zu transferieren, und dies auch global. So ist es möglich Kooperationen zwischen Anwendern auf der Basis valider Daten und rückführbarer Ergebnisse zu stützen. Damit ist die Möglichkeit geschaffen, die Zellkultivierung reproduzierbarer zu machen. Insbesondere bei schlechten Wachstumsergebnissen sind die Messwerte der Zellkultur allein nicht ausreichend, um eine Ursachenanalyse zu betreiben. Nun ist es erstmals möglich die Ursachenanalyse zu vereinfachen, indem auch die Kammerparameterdaten des Inkubators, also die Umgebungsbedingungsdaten, zur Verfügung stehen und bei der Ursachenanalyse einfließen können.

Das Innere einer geschlossenen Inkubatorkammer und die darin vorgesehenen Teile bilden ein physikalisches System, das im Optimalfall präzise vom Inkubator kontrollierbar ist. Dazu dienen insbesondere die präzise arbeitenden und vorzugsweise kalibrierbaren Sensoreinrichtungen des Inkubators. Dieses geschlossene System definiert die Lebensumgebung der Zellkulturen. Die Erfassung der physikalischen Zustände dieses geschlossenen Systems erlaubt somit eine genaue Erfassung der Lebens- bzw. Wachstumsumstände der Zellkulturen. Diese Konstellation bietet ideale Voraussetzungen, um den Inkubator als Datenerhebungszentrale zu verwenden. Insbesondere ist ein Vorteil dieser Konstellation, dass präzise Informationen über alle Maßnahmen und Vorgänge an der Inkubatorkammer vorliegen, die vom Inkubator bzw. dessen elektronischer Steuereinrichtung selbst gesteuert, also insbesondere initiiert werden.

Ein besonders relevanter, vom Inkubator gesteuerter, oder zumindest vorzugsweise von einem Sensor des Inkubators erfasster Vorgang ist das Öffnen und/oder Schließen einer Inkubatortüre, insbesondere einer Kammertüre der Inkubationskammer. Die Sensoreinrichtung ist vorzugsweise dazu eingerichtet, das Öffnen und/oder Schließen der Inkubatortüre als Kammerparameter zu erfassen, da es sich insbesondere um einen physikalischen Zustand der Inkubatorkammer handelt. Vorzugsweise wird dieser Kammerparameter jeweils zeitabhängig gespeichert. Die Information "Tür geöffnet" und/oder "Tür geschlossen", insbesondere zeitabhängig, kann in Form von Werten eines entsprechenden Inkubatortürparameters gespeichert werden. Erfahrungsgemäß beeinflusst das Öffnen der Inkubatortüre und die entsprechende Atmosphärenänderung im Inneren der Inkubatorkammer das Zellwachstum erheblich, besonders wenn dies öfters erfolgt - was bei einem mit mehreren Zellkulturen bestückten Inkubator in der Praxis wahrscheinlich ist. Es ist deshalb besonders nützlich, den Inkubatortürparameter mit einem Wachstumsparameter zu korrelieren, insbesondere wenn beide zeitabhängig gespeichert werden. Kammerparameter sind insbesondere die in der Inkubatorkammer vorliegenden atmosphärischen Bedingungen, insbesondere CO₂, O₂, N₂, relative Luftfeuchtigkeit, und/oder Anzahl bzw. Dauer der Türöffnungen.

Zu den vom Inkubator selbst gesteuerten Vorgängen gehören zudem alle Temperierschritte, die den physikalischen Zustand "Temperatur der Inkubatoratmosphäre" beeinflussen, insbesondere auch Schritte der automatischen Desinfektion mittels Hochtemperaturphasen bei ca. 100 °C bis 120 °C oder bis 180°C oder 200°C, die bei leerer Inkubatorkammer durchgeführt werden. Der Inkubator bzw. dessen Steuereinrichtung ist vorzugsweise dazu eingerichtet, die zu einem bestimmten Zeitpunkt als Solltemperatur eines Temperaturregelkreises angelegte Temperatur oder die infolge der Temperaturregelung detektierten Temperaturwerte in den Zellüberwachungsdaten zu speichern.

Zu den vom Inkubator selbst gesteuerten Vorgängen können ferner Gasaustauschvorgänge gehören, bei denen gemäß einem Volumenstrom mit einem vorbestimmten Volumen pro Zeit Teile der Inkubatoratmosphäre ausgetauscht werden, um insbesondere als Bestandteil eines Regelvorgangs die gewünschte Gaszusammensetzung der Inkubatoratmosphäre einzustellen, insbesondere wenn sich diese nach einem Öffnen der Inkubatortüre in unerwünschter Weise geändert hat. Der entsprechende zu messende physikalische Zustand ist in diesem Fall die relative Gaskonzentration oder die relative Luftfeuchtigkeit. Der Inkubator bzw. dessen Steuereinrichtung ist vorzugsweise dazu eingerichtet, die zu einem bestimmten Zeitpunkt als Sollwert eines Temperaturregelkreises angelegte die relative Gaskonzentration oder den infolge der relativen Gaskonzentration detektierten Gaswerte, insbesondere CO₂ und/oder O₂-Werte, in den Zellüberwachungsdaten zu speichern. Zu dieser Gasregelung lässt sich auch die Regelung der Luftfeuchtigkeit rechnen, die mittels eines Luftfeuchtesensors einer Sensoreinrichtung erfolgt, insbesondere mittels eines Sensors zur Messung der relativen Luftfeuchtigkeit.

Zu den vom Inkubator selbst gesteuerten Vorgängen können ferner Gasbewegungsvorgänge gehören, bei denen beispielsweise gemäß einem Volumenstrom mit einem vorbestimmten Volumen pro Zeit und insbesondere auch in einer oder mehrere variabler oder konstanter Strömungsrichtungen in der Inkubatorkammer Teile der Inkubatoratmosphäre bewegt werden. Dies kann insbesondere zu einer gleichmäßigeren Atmosphäre innerhalb der Inkubatorkammer führen, um insbesondere unterschiedlich positionierte Zellkulturbehälter im zeitlichen Mittel mit derselben Atmosphäre zu beaufschlagen.

Die Messeinrichtung ist zur Messung mindestens eines Wachstumsparameters eingerichtet, der das Wachstum der Zellen dieser Zellkultur charakterisiert. Der Wachstumsparameter kann der pH-Wert des Zellmediums der Zellkultur sein, wenn die Messeinrichtung mindestens einen pH-Detektor aufweist, mit dem der pH-Wert des Mediums messbar oder schätzbar ist. Der pH-Detektor kann non-invasiv sein, indem er beispielsweise auf einer optischen Messung des Zellmediums beruht, insbesondere auf einer Farberkennung des Zellmediums. Zellmedien werden mit pH-abhängigen Farbstoffen versehen, um die visuelle Inspektion des pH-Wertes zu ermöglichen. Dieser Umstand lässt sich auf die genannte Weise auch für eine optisch-apparative Messung nutzen.

Der Wachstumsparameter kann die Anzahl der Zellen in einem Testabschnitt des Zellkulturbehälters beschreiben, wenn die Messeinrichtung als Zell-Zähl-Messeinrichtung eingerichtet ist. Dazu weist die Messeinrichtung vorzugsweise einen bilderfassenden Sensor, z.B. einen CMOS-Sensor, auf, mittels dem ein Bild oder ein Film der zu zählenden Zellen erfasst wird. Unter einem Bild wird vorzugsweise ein Datensatz von Daten verstanden, die insbesondere Werte von Lichtintensitäten enthalten, die mittels der lichtsensitiven Elemente eines Bildsensors empfangen wurden. Im Rahmen der vorliegenden Erfindung kann, aber muss nicht zwingend, ein Bild eine optische Abbildung, insbesondere Abbildung einer Zellkultur, bezeichnen. Ein Bild kann dann durch eine software-basierte Bilderkennung ausgewertet werden, die vorzugsweise als Bestandteil in den Inkubator oder das System gemäß der Erfindung integriert ist, insbesondere durch eine Partikel-Erkennung und -Zählung. Ferner kann als Bestandteil der Messeinrichtung eine Lichtquelle vorgesehen sein, deren Strahlung vorzugsweise die Zellkultur erfasst und insbesondere auf diese gerichtet ist. Ferner kann als Bestandteil der Messeinrichtung mindestens ein Mittel zur Beeinflussung des von der Lichtquelle abgestrahlten Lichts und/oder des vom Sensor erfassten Lichts vorgesehen sein. Mittels der Optik kann eine Auflichtmikroskopie realisiert sein, insbesondere eine Phasenkontrast- oder Dunkelfeldmikroskopie.

Der Wachstumsparameter kann die Konfluenz des Zellrasens im Zellkulturbehälter beschreiben, also die Zelldichte. Dazu ist die Messeinrichtung vorzugsweise als Konfluenz-Messeinrichtung eingerichtet. Zu diesem Zweck weist die Messeinrichtung vorzugsweise einen optischen Sensor auf, und vorzugsweise eine Lichtquelle als Bestandteil der Messeinrichtung, deren Strahlung vorzugsweise die Zellkultur erfasst und insbesondere auf diese gerichtet ist. Ferner kann als Bestandteil der Messeinrichtung mindestens ein Mittel zur Beeinflussung des von der Lichtquelle abgestrahlten Lichts und/oder des vom Sensor erfassten Lichts vorgesehen sein. Diese Mittel sind aus der Gruppe der bevorzugten Mittel ausgewählt, beinhaltend eine Linse, ein Prisma, ein optischer Filter, ein Spiegelelement, eine lichtleitende Faser. Mittels der Optik kann eine Auflichtmikroskopie realisiert sein, insbesondere eine Phasenkontrast- oder Dunkelfeldmikroskopie. Dieser Sensor ist vorzugsweise ein ortsauflösender Sensor, z.B. ein CMOS-Sensor oder eine Kamera. Mit solch einem Sensor ist insbesondere ein Bild des Zellrasens erfassbar. Dieses Bild kann dann durch eine software-basierte Bilderkennung ausgewertet werden, insbesondere durch eine Kantendetektion und - Zählung der von Kanten umschlossenen Gebilde, die als adhärente Zellen des Zellrasens interpretierbar sind.

Die Messeinrichtung kann zudem dazu eingerichtet sein, die Konfluenz mittels einer elektrischen Messung am Zellrasen der Zellkultur durchzuführen, insbesondere mittels mindestens einer Messung des elektrischen Widerstands bzw. der Impedanz des wachsenden Zellrasens, insbesondere mittels zeitaufgelöster Messungen. Der Zellrasen bildet eine elektrische Kapazität, die zu seiner Charakterisierung verwendbar ist. Dazu weist ein Zellkulturgefäß vorzugsweise eine Elektrode auf, insbesondere planare Elektrode, oberhalb der sich der Zellrasen bildet. Diese Elektrode ist mit der Messeinrichtung des Inkubators elektrisch verbindbar ausgebildet. Eine solche Messtechnik ist insbesondere unter der Bezeichnung Impedimetrie bzw. ECIS (englisch: electric cell-substrate impedance sensing) bekannt.

Andere Verfahren zur Bestimmung des Konfluenzstadiums des Zellrasens auf dem Substrat eines Zellkulturbehälters können beinhalten, dass die Zellen radioaktiv markiert, und/oder mittels Zellfärbung markiert und mittels Auflichtmikroskopie, insbesondere Phasenkontrast- oder Dunkelfeldmikroskopie betrachtet werden. Fluoreszenzmikroskopie zur Konflueszenzanalyse ist ebenfalls möglich. Dabei werden Zellen mit Fluoreszenz-markierten Proteinen/ Partikeln markiert und auf diese Weise sichtbar gemacht.

Der Wachstumsparameter kann zudem die Morphologie der Zellen des Zellrasens beschreiben. Die Zellmorphologie kann für bestimmte Wachstumsphasen der Zelle charakteristisch sein - nicht adhärente Zellen in Suspension sind meist sphärisch geformt während sie sich bei Adhäsion an ein planares Substrat abflachen und sich substratseitig im Wesentlichen planar ausbilden. Die Zellmorphologie kann für eine Zellart bzw. Zelllinie charakteristisch sein und mittels Morphologie-Messung identifizierbar sein. Die Zellmorphologie kann zudem einen apoptotischen oder nekrotischen Zustand der Zelle beschreiben, der mittels Morphologie-Messung spezifizierbar sein kann.

Deshalb ist die Messeinrichtung vorzugsweise als Morphologie-Messeinrichtung eingerichtet. Zu diesem Zweck weist die Messeinrichtung vorzugsweise einen optischen Sensor auf, und vorzugsweise eine Lichtquelle als Bestandteil der Messeinrichtung, deren Strahlung vorzugsweise die Zellkultur erfasst und insbesondere auf diese gerichtet ist. Ferner kann als Bestandteil der Messeinrichtung mindestens ein Mittel zur Beeinflussung des von der Lichtquelle abgestrahlten Lichts und/oder des vom Sensor erfassten Lichts vorgesehen sein. Diese Mittel sind aus der Gruppe der bevorzugten Mittel ausgewählt, beinhaltend eine Linse, ein Prisma, ein optischer Filter, ein Spiegelelement, eine lichtleitende Faser. Mittels der Optik kann eine Auflichtmikroskopie realisiert sein, insbesondere eine Phasenkontrast- oder Dunkelfeldmikroskopie. Dieser Sensor ist vorzugsweise ein ortsauflösender Sensor, z.B. ein CMOS-Sensor oder eine Kamera. Mit solch einem Sensor ist insbesondere ein Bild des Zellrasens erfassbar. Dieses Bild kann dann durch eine software-basierte Bilderkennung ausgewertet werden, insbesondere durch eine Kantendetektion und -Zählung der von Kanten umschlossenen Gebilde, die als adhärente Zellen des Zellrasens interpretierbar sind.

Die Messeinrichtung weist vorzugsweise einen Analog-Digital-Wandler auf, um den mindestens einen zweiten Messwert, also die Messwerte des mindestens einen Wachstumsparameters, zu digitalisieren und als Zellüberwachungsdaten bereitzustellen. Die Messeinrichtung weist vorzugsweise eine (eigene) Datenverarbeitungseinrichtung auf, insbesondere einen Mikroprozessor, die insbesondere auch als zweite Datenverarbeitungseinrichtung bezeichnet wird, und vorzugsweise eine eigene Datenspeichereinrichtung, die insbesondere auch als zweite Datenspeichereinrichtung bezeichnet wird. Die Messeinrichtung ist vorzugsweise dazu eingerichtet, den mindestens einen zweiten Messwert unabhängig von der Datenverarbeitungseinrichtung des Inkubators zu messen bzw. die Durchführung dieser Messung zu steuern, insbesondere gemäß einem eigenen Steuerprogramm (zweites Steuerprogramm), das von der zweiten Datenverarbeitungseinrichtung durchgeführt wird.

Es ist aber auch möglich und besonders bevorzugt, dass die Datenverarbeitungseinrichtung des Inkubators (erste Datenverarbeitungseinrichtung) die Durchführung der Messung des mindestens einen zweiten Messwertes steuert. Dadurch lässt sich eine energiearme und effiziente Erfassung von Zellüberwachungsdaten realisieren. Es ist bei der Umsetzung zu beachten, dass der Betrieb von elektrischen Einrichtungen innerhalb der Inkubatorkammer eventuell zu Abwärme führt, welche die Kammeratmosphäre in inakzeptablem Maße erwärmt. Die Steuereinrichtung des Inkubators kann deshalb insbesondere dazu eingerichtet sein, den Betrieb der elektrischen Einrichtungen, insbesondere der mindestens einen Messeinrichtung und/oder einer Behandlungseinrichtung innerhalb der Inkubatorkammer in Abhängigkeit von mittels Temperatursensoren erfassten Temperaturen der Kammeratmosphäre zu steuern. Die Steuereinrichtung des Inkubators kann insbesondere dazu eingerichtet sein, die Temperierung der Kammeratmosphäre mittels der mindestens einen Temperiereinrichtung und den Betrieb der elektrischen Einrichtungen innerhalb der Inkubatorkammer in Abhängigkeit voneinander zu steuern, um die unerwünschte Erwärmung der Kammeratmosphäre zu kompensieren.

Die Erfassung der ersten und zweiten Messwerte und/oder deren Speichern erfolgt vorzugsweise gesteuert durch die Datenverarbeitungseinrichtung bzw. die Steuereinrichtung des Inkubators, insbesondere gemäß den Vorgaben eines Überwachungsprogramms. Das Überwachungsprogramm kann Bestandteil des Steuerungsprogramms des Inkubators sein, insbesondere ein Unterprogramm.

Die Datenverarbeitungseinrichtung ist vorzugsweise Bestandteil einer Steuereinrichtung des Inkubators, die Funktionen des Inkubators steuert. Die Funktionen der Steuereinrichtung sind insbesondere durch elektronische Schaltkreise implementiert. Die Steuereinrichtung kann einen Mikroprozessor aufweisen, der die Datenverarbeitungseinrichtung beinhalten kann. Die Steuereinrichtung und/oder die Datenverarbeitungseinrichtung ist vorzugsweise zur Durchführung eines Steuerungsverfahrens ausgebildet, das auch als Steuerungssoftware oder Steuerungsprogramm bezeichnet wird. Die Funktionen des Inkubators und/oder der Steuereinrichtung können in Verfahrensschritten beschrieben werden. Sie können als Bestandteile des Steuerungsprogramms realisiert sein, insbesondere als Unterprogramme des Steuerungsprogramms.

Eine Steuereinrichtung weist im Rahmen der vorliegenden Erfindung generell insbesondere die Datenverarbeitungseinrichtung, insbesondere eine Recheneinheit (CPU) zum Verarbeiten von Daten und/oder einen Mikroprozessor auf oder ist die Datenverarbeitungseinrichtung. Die Datenverarbeitungseinrichtung der Steuereinrichtung des Inkubators ist vorzugsweise auch zum Steuern eines Behandlungsprozesses und/oder von individuellen Behandlungen eingerichtet, die von einer oder mehreren insbesondere optionalen Behandlungseinrichtungen des Inkubators durchgeführt werden.

Die Datenverarbeitungseinrichtung ist alternativ vorzugsweise eine außerhalb des Inkubators und getrennt von diesem angeordnete Vorrichtung, auch als externe Vorrichtung bzw. externe Datenverarbeitungseinrichtung bezeichnet. Die Datenverarbeitungseinrichtung und der Inkubator stehen vorzugsweise in einer Datenverbindung und sind vorzugsweise Bestandteile eines Netzwerks zum Datenaustausch. Die Datenverarbeitungseinrichtung und der Inkubator sind in diesem Fall insbesondere Bestandteile eines erfindungsgemäßen Systems zur Überwachung des Wachstums von Zellkulturen. Solche Netzwerkbestandteile werden hier auch als Netzwerkinstrumente bezeichnet. Auch die mindestens eine Messeinrichtung kann als Netzwerkinstrument eingerichtet sein. Das Netzwerk zum Datenaustausch ist vorzugsweise ein im Wesentlichen nicht-hierarchisches Netzwerk, insbesondere ein Netzwerk, dessen Teilnehmer, also Netzwerkinstrumente, zumindest teilweise oder alle eine Peer-to-Peer-Netzwerkarchitektur bilden. In einem Peer-to-Peer-Netzwerk sind alle Teilnehmer gleichberechtigt und können sowohl Dienste in Anspruch nehmen, als auch zur Verfügung stellen. Das Netzwerk zum Datenaustausch verwendet vorzugsweise eine Ethernet Technologie und ist vorzugsweise ein Ethernet-Netzwerk.

Das System, der Inkubator und/oder die Steuereinrichtung und/oder die Datenverarbeitungseinrichtung und/oder die mindestens eine Messeinrichtung sind vorzugsweise dazu eingerichtet, die Zellüberwachungsdaten gemäß mindestens einer der nachstehend genannten Konfigurationen zu erfassen:
- Es werden Zellüberwachungsdaten zu Zeitpunkten gemäß einem vordefinierten Zeitplan erfasst, der vom Inkubator festgelegt worden sein kann und/oder vom Benutzer über eine Benutzerschnittstelleneinrichtung des Inkubators beeinflusst oder festgelegt worden sein kann; solche Angaben des Zeitpunkts können absolute Datums- und Uhrzeitangaben enthalten, die der Inkubator durch Vergleich mit den Daten einer ihm optional und vorzugsweise verfügbaren Uhr verwertet;
- Es werden Zellüberwachungsdaten in vorgegebenen Zeitabständen, insbesondere regelmäßigen Zeitabständen, z.B. Zeitabstände dt = 10 Minuten bis 20 Minuten u.s.w., erfasst, die vom Inkubator festgelegt worden sein können und/oder vom Benutzer über eine Benutzerschnittstelleneinrichtung des Inkubators beeinflusst oder festgelegt worden sein können;
- Es werden Zellüberwachungsdaten in Abhängigkeit von mindestens einem vorgegebenen Ereignis erfasst, das vorzugsweise aus der Gruppe der folgenden Ereignisse ausgewählt ist:
   ∘ ein mittels der Sensoreinrichtung gemessener erster Messwert erfüllt eine vorgegebene Bedingung, z.B. eine unerlaubte Abweichung von der Solltemperatur (37 °C) in der Inkubatorkammer, die z.B. durch eine geöffnete Inkubatortüre verursacht sein kann, oder eine unerlaubte Abweichung von einer relativen Gaskonzentration in der Inkubatorkammer, insbesondere ein CO2 und/oder O2-Wert und/oder N2-Wert; ein typischer CO2-Wert in Inkubatorkammern liegt bei 5%;
   ∘ ein vom Inkubator erkannter Fehlerzustand des Inkubators, betreffend Hardware oder Software;
   ∘ Es wird über eine Kommunikationseinrichtung der Steuereinrichtung des Inkubators oder der Messeinrichtung ein Steuersignal empfangen, das die Erfassung von Zellüberwachungsdaten auslöst; ein solches Steuersignal kann insbesondere von einer externen Datenverarbeitungseinrichtung veranlasst sein oder durch eine Benutzereingabe veranlasst worden sein;
- Ein von der Datenverarbeitungseinrichtung oder der Steuereinrichtung des Inkubators oder der Messeinrichtung ausgeführtes Steuerprogramm löst eine Messung von Zellüberwachungsdaten aus, nachdem ein vom Steuerprogramm angewandtes Auswertungsverfahren zuvor gemessene zweite Messwerte ausgewertet hat; dabei kann das Auswertungsprogramm die Auslösung der Messung von Zellüberwachungsdaten aufgrund einer erfüllten Bedingung vorsehen, diese Bedingung kann aus der Gruppe der folgenden Bedingungen ausgewählt sein:
   ∘ Mindestens ein zuvor gemessener zweiter Messwert weicht von mindestens einem Referenzwert ab oder liegt außerhalb eines vordefinierten Bereichs;
   ∘ Mindestens ein Verlauf aus zwei oder vielen zuvor gemessenen zweiten Messwerten weicht von mindestens einem Referenzverlauf ab oder liegt außerhalb eines vordefinierten Referenzbereichs; dieser Referenzverlauf oder Referenzbereich kann ein bekannter Normverlauf oder Normbereich sein, der insbesondere von anderen Parametern abhängen kann, insbesondere der Information über die Zellart und/oder das für die betreffende Zellkultur verwendete Zellmedium.

Das System, der Inkubator und/oder die Steuereinrichtung und/oder die Datenverarbeitungseinrichtung und/oder die mindestens eine Messeinrichtung sind vorzugsweise dazu eingerichtet, die Zellüberwachungsdaten in Abhängigkeit von Zellidentifikationsdaten zu erfassen. Zellidentifikationsdaten können zuvor vom Benutzer zum Beispiel über eine Benutzerschnittstelleneinrichtung des Inkubators eingegeben worden sein. Der Inkubator oder das System kann auch eine Identifikationseinrichtung zur Erkennung eines Zellkulturbehälters oder einer Zellkultur beinhalten. Insbesondere können dem Inkubator Korrelationsdaten Informationen über die Korrelation aus Zellidentifikationsdaten und einer zuvor erstellten Markierung vorliegen. Die Markierung kann am Zellkulturbehälter angebracht sein und kann über eine Leseeinrichtung des Inkubators oder des Systems maschinen-lesbar sein. Die Markierung kann ein Code sein, insbesondere ein Barcode, ein zweidimensionaler Code, oder ein maschinenlesbares Zeichen oder eine Zeichenfolge.

Durch die Erfassung von Zellüberwachungsdaten in Abhängigkeit von Zellidentifikationsdaten kann eine Datensammlung in Abhängigkeit von einer Zellart, insbesondere eine Zellinie angelegt und gespeichert werden. Auf diese Weise lässt sich insbesondere die Wachstumshistorie einer bestimmten Zellprobe bzw. Zellinienprobe und deren durch Passagieren erhaltenen Subkulturen erfassen. Es kann eine elektronische Zellakte mit diesen historischen Wachstumsdaten erfasst werden.

Unabhängig von der Entwicklung einer einzelnen Zellprobe bzw. Zellinienprobe, die eventuell passagiert, eingefroren und wieder aufgetaut wurde, kann auch das Wachstumsverhalten einer Zellart bzw. Zelllinienart verfolgt werden, indem Zellüberwachungsdaten dieser Zellart bzw. Zelllinienart aus mehreren einzelnen Zellproben bzw. Zellinienproben wiederholt erfasst werden, vorzugsweise weiter in Abhängigkeit vom verwendeten individuellen Inkubator, einer Fertigungsserie eines Inkubators, einem Inkubatortyp oder anderen Informationen. Diese Erfassung kann im erfindungsgemäßen System insbesondere über regional oder global verteilte Inkubatoren erfolgen, bzw. landesweit und weltweit. Auf diese Weise lässt sich eine elektronische Zellakte aus diesen historischen Wachstumsarten mit Bezug auf eine Zellart bzw. Zelllinienart erstellen. Daraus lassen sich wertvolle Referenzdaten und Informationskorrelationen ableiten, insbesondere Normverläufe und Normbereiche festlegen, die wiederum vom erfindungsgemäßen Inkubator oder System als Referenzdaten verwendbar sind.

Informationskorrelationen können z.B. beinhalten, wie eine bestimmte Zelllinie ab einer bestimmten Zahl von Passagiervorgängen ein anderes charakteristisches Wachstum besitzt als davor bzw. wie das Wachstumsverhalten von der Nummer des Passagierens abhängt. Informationskorrelationen können z.B. auch beinhalten, wie das Wachstumsverhalten einer bestimmten Zelllinie von der Anzahl der Öffnungen der Inkubatortür oder der Gesamtöffnungszeit der Inkubatortür oder einer anderen statistischen Information über die Öffnung der Inkubatortür abhängt. Informationskorrelationen können z.B. auch beinhalten, wie das Wachstumsverhalten einer bestimmten Zelllinie von der Anzahl der und/oder Art der Wechsel des Nährmediums der Zellkultur abhängt. Informationskorrelationen können z.B. auch beinhalten, wie der Typ des Zellkulturbehälters, in dem die Zellen wachsen, sich auf das Wachstumsverhalten auswirkt. Der Inkubator könnte zu diesem Zweck vom Benutzer die Information über den verwendete Typ des Zellkulturbehälters oder eine individuelle Identifikationsnummer des Zellkulturbehälters über die Erfassung der Zellkulturbehälterdaten an einer Benutzerschnittstelle erfassen. Auch eine Bestell- und Lotnummer kann hier mit-erfasst werden. Somit kann insbesondere rückverfolgt werden, ob die Wachstumsoberfläche des Zellkulturbehälters oder des Typs oder einer Herstellungscharge dieses Typs einwandfrei funktionierte.

Insbesondere kann vom erfindungsgemäßen Inkubator oder System in Abhängigkeit von solchen Referenzdaten mindestens ein Steuerparameter festgelegt werden, der das Steuerungsverfahren des Inkubators beeinflusst. Diese Beeinflussung kann darin bestehen, dass das Steuerungsverfahren dem Benutzer in Abhängigkeit vom Steuerparameter über eine Benutzerschnittstelleneinrichtung eine Information darüber ausgibt, zu welchem Zeitpunkt der Zellkulturbehälter aus dem Inkubator entfernt werden muss, damit ein bestimmter vom Benutzer gewünschter Wachstumsparameter vorliegt, z.B. Adhäsion der Zellen, Zellzahl im Behälter, Zelldichte, Morphologie.

Das System, der Inkubator und/oder die Steuereinrichtung und/oder die Datenverarbeitungseinrichtung und/oder die mindestens eine Messeinrichtung sind vorzugsweise dazu eingerichtet, die Zellüberwachungsdaten zur Bildung einer elektronischen Dokumentationsdatei zu verwenden, in der das Wachstum einer individuellen Zellprobe in einem Zellkulturbehälter oder das Wachstum von mehreren Zellkulturen in Zellkulturbehältern protokolliert und dokumentiert wird. Diese Dokumentationsdatei wird dann in einer Datenspeichereinrichtung gespeichert. Da die Zellüberwachungsdaten Rückschlüsse auf die Umgebungsbedingungen erlauben, kann einerseits die Einhaltung bestimmter Standardprotokolle für das Kultivieren von Zellen zertifiziert werden. Andererseits können nachträglich Abweichungen von solchen Standartprotokollen ermittelt werden und/oder Informationskorrelationen ermittelt werden. Durch die Erfassung solcher Daten kann die Qualität von zellbasierten Laborarbeiten bzw. medizinischen, biologischen und pharmazeutischen Verfahren erheblich verbessert werden und zuverlässiger werden. Die Reproduzierbarkeit der zellbasierten Laborarbeiten kann erhöht werden, Abweichungen von normalen Eigenschaften können frühzeitige erkannt werden, um dem Anwender die Möglichkeit einer Korrektur oder frühzeitigen Wiederholung des Experiments zu geben. Die Dokumentationsdatei kann dem Benutzer oder einer externen Datenverarbeitungseinrichtung von der Steuerungseinrichtung über Datenaustausch bereitgestellt werden. Insbesondere in kritischen Anwendungen, die z.B. einen forensischen Bezug haben oder bei denen Zellen erheblichen Wertes kultiviert werden, ist eine solche Dokumentation besonders sinnvoll.

Die Erfindung dient insbesondere dazu, eine Standardisierung des Wachstumsverhaltens der Zellen in Zellkulturen zu erzielen, damit der Benutzer das von ihm gewünschte Wachstumsergebnis erreicht, insbesondere eine vorgegebene Zelladhäsion, wie z.B. nach dem Auftauen von Zellen, Anzahl von Zellen in Zellkultur, Zelldichte und/oder Morphologie der Zellen, jeweils auch als Wachstumsziel bezeichnet. Dies wird insbesondere dadurch erreicht, dass die Abhängigkeit des Zellwachstums von ersten Messwerten und/oder zweiten Messwerten, insbesondere von Zellüberwachungsdaten, erfasst wird. Das Zellwachstum wird insbesondere durch mindestens einen Wachstumsparameter repräsentiert. Insbesondere das zeitweise Erfassen der zweiten Messwerte kann unter Verwendung eines Planungsprogramms dazu verwendet werden, das Wachstumsziel zu erreichen. Der erfindungsgemäße Inkubator dient insbesondere als Werkzeug einer Vereinheitlichung (Standardisierung) des Vorgehens zum Erreichen gewünschter Wachstumsparameter bei Zellkulturen. Die Zellüberwachungsdaten bilden eine Datenbasis, anhand der Korrelationen von Zellüberwachungsdaten, insbesondere von ersten und zweiten Messwerten ermittelbar sind (Informationskorrelationen), oder Kausalzusammenhänge zwischen Zellüberwachungsdaten, insbesondere in Abhängigkeit von einer Zellart, insbesondere Zelllinie. Beispielsweise kann ermittelt werden, wie ein Wachstumsparameter von einem Inkubatortürparameter abhängt. Die so ermittelte Korrelation oder der so ermittelte Zusammenhang kann zur Festlegung eines Planungsprogramms für eine Zellkultur festgelegt werden. Beispielsweise kann festgestellt werden, dass -insbesondere bei einer bestimmten Zellart- die Zellkonfluenz nach dem Aussähen der Zellen und/oder dem Start des Inkubierens abhängig ist von einer bestimmten Anzahl des Öffnens der Inkubatortüre in einem definierten Zeitraum ab dem Aussähen der Zellen und/oder dem Start des Inkubierens, insbesondere dass das Zellwachstum durch dieses Türöffnen verlangsamt wird. Aus diesem Zusammenhang lässt sich zum Beispiel schlussfolgern, dass der Anwender erst ab einem bestimmten, vom Inkubator aus der bekannten Korrelation errechneten Zeitpunkt ab dem Aussähen der Zellen und/oder dem Start des Inkubierens mit dem Erreichen der gewünschten Konfluenz rechnen kann.

Der Inkubator und/oder die Steuereinrichtung und/oder die Datenverarbeitungseinrichtung sind vorzugsweise dazu eingerichtet, mindestens einen Arbeitsschritt in Abhängigkeit von Zellüberwachungsdaten durchzuführen. Diese Ausgestaltung erweitert die Funktionalität in vorteilhafter Weise. In einer bevorzugten Gestaltung beinhaltet dieser Arbeitsschritt, dem Benutzer mittels einer Benutzerschnittstelleneinrichtung eine Information über die Zellüberwachungsdaten auszugeben oder Zellüberwachungsdaten in einer Datenspeichereinrichtung abzuspeichern.

Vorzugsweise beinhaltet das Steuerungsprogramm des Inkubators oder des Systems oder einer externen Datenverarbeitungseinrichtung ein Planungsprogramm, insbesondere Zeitplanungsprogramm, mittels dem ein Wachstumsplan in Form von Planungsdaten in Abhängigkeit von Zellüberwachungsdaten errechnet werden kann. Dieser Wachstumsplan kann mittels der Zellüberwachungsdaten mindestens einen Wachstumsparameter errechnen, insbesondere den zeitlichen Verlauf desselben. Es kann auch der Zeitpunkt oder der Zeitabschnitt errechnet werden, an dem/nach dem ein Wachstumsparameter eine vorgegebene Bedingung, z.B. einen vorgegebenen Wert erfüllt. Dieser Wachstumsparameter kann insbesondere sein: eine Zelladhäsion (z.B. Anteil der Zellen im Zellkulturbehälter, die am Substrat des Zellkulturbehälters adhäriert sind), eine Anzahl von nach Zellteilungen vorhandener Zellen im Zellkulturbehälter, eine Zelldichte (Anzahl der nach Zellteilungen vorhandener Zellen im Zellkulturbehälter pro Substratfläche), Verdopplungszeit, oder Zellmorphologie (z.B. statistische Informationen über im Zellkulturbehälter vorhandene Zellen mit bestimmten morphologischen Eigenschaften wie die absolut durch eine Zelle belegter Substratfläche, Formkontur- z.B. Kugelformähnlichkeit, Höhe im vertikalen Abstand zum Substrat etc., ermittelbar insbesondere jeweils über Bilderfassung und Bildauswertung). Der Wachstumsparameter kann ferner den pH-Wert des Zellnährmediums enthalten, der z.B. über eine optische Absorptionsmessung am Zellnährmedium ermittelt wurde.

Es kann insbesondere mittels Planungsprogramm auch errechnet werden, zu welchen Zeiten eine elektronische Zugriffssperreinrichtung des Inkubators den Zugriff des Benutzers auf mindestens eine Funktion des Inkubators automatisch verhindert. Eine Inkubatortüre kann als Zugriffssperreinrichtung insbesondere ein elektronisch ansteuerbares Schloss aufweisen, durch das das Öffnen einer Inkubatortüre verhindert wird. Die Zugriffssperreinrichtung kann insbesondere Bestandteil eines Steuerprogramms sein, durch das eine Software-gesteuerte Funktion des Inkubators aktivierbar/deaktivierbar ist. Auf diese Weise lässt sich das Wachstum der Zellkultur im Inkubator in empfindlichen Phasen vor einer atmosphärischen Störung schützen.

Vorzugsweise beinhaltet das Steuerungsprogramm des Inkubators oder des Systems oder einer externen Datenverarbeitungseinrichtung mindestens einen Vorhersagealgorithmus, um mittels der Zellüberwachungsdaten mindestens einen Wachstumsparameter errechnen, insbesondere den zeitlichen Verlauf desselben. Der Vorhersagealgorithmus beschreibt vorzugsweise, dass sich der bekannte Normverlauf eines Wachstumsparameters in Abhängigkeit von Zellüberwachungsdaten verändert.

Dem in Abhängigkeit von Zellüberwachungsdaten durchgeführten Arbeitsschritt kann ein Vergleich der Zellüberwachungsdaten mit Referenzdaten als Bestandteil eines Auswertungsverfahrens vorausgehen. Diese Referenzdaten können zuvor von demselben Inkubator ermittelt worden sein oder per Datenverbindung an den auswertenden Inkubator übertragen worden sein.

Der in Abhängigkeit von Zellüberwachungsdaten durchgeführte Arbeitsschritt kann vorsehen, dem Benutzer über eine Benutzerschnittstelleneinrichtung Informationen, insbesondere betreffend Planungsdaten, auszugeben oder über eine Kommunikationseinrichtung des Inkubators Daten, insbesondere Planungsdaten, an eine externe Datenverarbeitungseinrichtung zu übersenden.

Der in Abhängigkeit von Zellüberwachungsdaten durchgeführte Arbeitsschritt kann vorsehen, dass die Steuerung mindestens eines Steuerungsparameters betreffend die Einstellung bzw. Regelung der Atmosphäre in der Inkubatorkammer in Abhängigkeit von Zellüberwachungsdaten erfolgt. Der in Abhängigkeit von Zellüberwachungsdaten durchgeführte Arbeitsschritt kann vorsehen, dass die Steuerung einer Behandlung mittels einer Behandlungseinrichtung in Abhängigkeit von Zellüberwachungsdaten erfolgt.

Das Steuerungsverfahren ist vorzugsweise dazu eingerichtet, den Inkubator in Abhängigkeit von Zellüberwachungsdaten so zu betreiben, dass ein vorgegebenes Wachstumsziel erreicht wird.

Der Inkubator ist ein Labor-Inkubator und damit ein Gerät, mit dem kontrollierte Klimabedingungen für verschiedene biologische Entwicklungs- und Wachstumsprozesse geschaffen und erhalten werden können. Er dient insbesondere der Schaffung und Erhaltung eines Mikroklimas mit geregelten Gas-, und/oder Luftfeuchtigkeits- und/oder Temperatur-Bedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des CO₂ und/oder des O₂ und/oder des N2-Gehalts Gehalts des Gases und/oder eine Einstelleinrichtung zur Einstellung der Luftfeuchtigkeit in der Inkubatorkammer des Inkubators. Der Inkubator, insbesondere eine Behandlungseinrichtung des Inkubators, weist insbesondere die Inkubatorkammer auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. Je nach Ausführungsform kann darüber auch die Luftfeuchte geregelt werden. Eine mit Wasser gefüllte Wanne in der Inkubatorkammer kann geheizt oder gekühlt werden, um über die Verdunstung die Luftfeuchtigkeit einzustellen. CO₂-Inkubatoren dienen insbesondere der Kultivierung tierischer bzw. humaner Zellen. Inkubatoren können Wendevorrichtungen zum Wenden des mindestens einen Zellkulturbehälters und/oder eine Schütteleinrichtung zum Schütteln bzw. Bewegen der des mindestens einen Zellkulturbehälters aufweisen.

Die Steuerungseinrichtung kann dazu eingerichtet sein, dass ein Programmparameter oder ein Steuerungsparameter des Inkubators automatisch in Abhängigkeit von Zellüberwachungsdaten gewählt wird. Eine von einem Steuerungsparameter gesteuerte Behandlung der mindestens einen Zellkultur in mindestens einem Zellkulturbehälter entspricht bei einem Inkubator insbesondere einer Klimabehandlung, der die mindestens eine Zellkultur unterzogen wird. Mögliche Parameter, insbesondere Programmparameter, insbesondere Nutzerparameter, die zur Beeinflussung einer Klimabehandlung verwendet werden, definieren insbesondere die Temperatur des Inkubatorraums, in dem die mindestens eine Probe inkubiert wird, die relative Gaskonzentration von O₂- und/oder CO₂ und/oder N₂ im Inkubationsinnenraum, die Luftfeuchtigkeit im Inkubationsinnenraum und/oder mindestens einen Ablaufparameter, der den Ablauf, insbesondere die Reihenfolge, eines aus mehreren Schritten bestehenden Inkubationsbehandlungsprogramms beeinflusst oder definiert.

Eine Sensoreinrichtung weist insbesondere mindestens einen Temperatursensor auf, vorzugsweise eine Vielzahl von Temperatursensoren. Ein Temperatursensor kann beispielsweise ein Pt 100- oder Pt 1000-Temperaturfühler sein. Eine Sensoreinrichtung weist vorzugsweise einen Sensor zur Ermittlung einer relativen Gaskonzentration auf, insbesondere zur Ermittlung des Gehalts an CO₂ und/oder O₂ und/oder N₂. Eine Sensoreinrichtung weist vorzugsweise einen Sensor zur Ermittlung der relativen Luftfeuchtigkeit auf.

Die Inkubatorkammer weist Kammerwände und mindestens eine Öffnung auf, über die die Zellkulturbehälter im Inneren der Inkubatorkammer platzierbar und entnehmbar sind. Diese Kammeröffnung ist durch ein mit der Inkubatorkammer beweglich verbundenes Verschlusselement verschliessbar, insbesondere eine mittels Scharnier an der Inkubatorkammer beweglich montierte Inkubatortüre, insbesondere eine oder mehrere Kammertüren. In der verschlossenen Position der Kammeröffnung ist das Innere der Inkubatorkammer gegen die Umgebung vorzugsweise in der Weise isoliert, so dass im Inneren eine gewünschte, vom Inkubator gesteuerte Atmosphäre einstellbar, insbesondere regelbar ist. In der geöffneten Position der Kammeröffnung ist über diese Öffnung ein Gasaustausch zwischen der Umgebung des Inkubators und dem Inneren der Inkubatorkammer möglich. Die Kammeröffnung befindet sich typischer Weise in der Frontwand der Inkubatorkammer.

Die Inkubatorkammer weist vorzugsweise mehrere Wände auf, die insbesondere einstückig und insbesondere kantenfrei miteinander verbunden sein können. Die Wände sind vorzugsweise im Wesentlichen planar geformt, können aber auch alle oder zum Teil eine geschwungene Form aufweisen. Die Inkubatorkammer ist vorzugsweise quaderartig geformt, kann aber auch anders geformt sein, z.B. sphärisch, ellipsoid, polyederförmig. Die Wände sind vorzugsweise aus einem korrosionsarmen Material gefertigt, insbesondere Edelstahl, Kupfer, Messing, oder ein Kunststoff, insbesondere ein Verbundkunststoff. Dadurch wird die Reinigung/ Desinfektion des Kammerinneren erleichtert. Unabhängig von der Öffnung, die dem Bestücken / Entnehmen von Zellkulturbehältern dient, kann die Inkubatorkammer einen Port zum Durchführen einer Kabelverbindung aus dem Inneren der Inkubatorkammer an dessen Außenseite oder in die Umgebung des Inkubators aufweisen.

Der Inkubator kann genau eine Inkubatorkammer aufweisen, kann aber auch mehrere Inkubatorkammern aufweisen, deren Atmosphäre (Temperatur, relative Gaskonzentration, Luftfeuchte) insbesondere individuell oder gesammelt einstellbar sein kann. Eine typische Größe des Inneren einer Inkubatorkammer liegt zwischen 50 und 400 Litern.

Die Inkubatorkammer kann einen Haltrahmen zum Halten eines oder mehrere Regalblecheinsätze oder einsetzbarer Instrumente aufweisen. Alternativ oder zusätzlich kann mindestens eine der Innenwände der Inkubatorkammer zum Halten eines oder mehrerer Regalblecheinsätze oder einsetzbarer Instrumente eingerichtet sein. Dazu kann eine in die Wand integrierte Haltestruktur vorgesehen sein, insbesondere ein oder mehrere Vorsprünge, Rinnen oder Stege. Ein Regalblecheinsatz vergrößert die Ablagefläche in der Inkubatorkammer. Ein in das Innere einsetzbares Instrument kann als Modul ausgebildet sein und ermöglicht die Durchführung von automatisierten Arbeitsschritten im Inneren, vorzugsweise auch bei geschlossener Inkubatortüre. Der Halterahmen ist ebenfalls vorzugsweise aus einem nicht-korrosiven Material gefertigt, vorzugsweise Edelstahl. Der Halterahmen ist vorzugsweise als stehendes Objekt ausgelegt, indem er mindestens einen Sockelabschnitt aufweist, der auf der Bodenwand der Inkubatorkammer ruht. Er kann sich aber auch an den Seitenwänden der Inkubatorkammer abstützen und/oder an der Deckenwand der Inkubatorkammer aufgehängt sein.

Der Inkubator kann ein Gehäuse aufweisen, dass die Inkubatorkammer teilweise oder vollständig umgibt. Das Gehäuse kann im Wesentlichen quaderförmig ausgebildet sein, und kann insbesondere derart ausgebildet sein, dass der Inkubator stapelbar ist.

Vorzugsweise weist der Inkubator eine Behandlungseinrichtung zur Behandlung des mindestens einen Zellkulturbehälters auf. Der Begriff "Behandlung" bedeutet insbesondere, dass eine Zellkultur oder ein Zellkulturbehältnis bewegt, und/oder transportiert und/oder untersucht und/oder verändert wird, insbesondere physikalisch, chemisch, biochemisch oder auf andere Weise verändert wird.

Eine Behandlungseinrichtung kann eine Bewegungseinrichtung sein, mittels der das Zellmedium in mindestens einem Zellkulturbehälter in Bewegung gehalten wird, vorzugsweise über ein Bewegungsprogramm, das vom Steuerprogramm gesteuert wird. Eine Bewegungseinrichtung kann eine Schüttel- oder Schwenkeinrichtung sein. Eine Bewegungseinrichtung weist vorzugsweise eine Trägereinrichtung auf, insbesondere eine Platte, auf der ein oder mehrere Zellkulturbehälter abgestellt und/oder fixiert werden. Eine Bewegungseinrichtung weist vorzugsweise eine Antriebseinrichtung auf, insbesondere im Fall einer Schütteleinrichtung beispielsweise einen Oszillatorantrieb, mittels dem das gewünschte Bewegungsprogramm umgesetzt wird. Die Gestaltung des Bewegungsprogramms kann vom Wachstumsstadium der Zellen einer Zellkultur abhängen und kann von der Zellart, insbesondere einer Zelllinie abhängen. Die Gestaltung und/oder Steuerung der Behandlung, insbesondere des Bewegungsprogramms, kann von den Zellüberwachungsdaten abhängen. Die Eine Behandlungseinrichtung kann eine Schwenkeinrichtung sein, mittels der mindestens ein Zellkulturbehälter geschwenkt wird. Die Bestandteile der Schwenkeinrichtung können denen der Schütteleinrichtung entsprechen, sind aber für eine Schwenkbewegung eingerichtet.

Eine Behandlungseinrichtung kann auch eine Transporteinrichtung sein, mittels der mindestens ein Zellkulturbehälter in der Inkubatorkammer transportierbar ist. Die Transporteinrichtung kann eine Lifteinrichtung sein, aufweisend eine Trägereinrichtung, auf der der mindestens eine Zellkulturbehälter platzierbar ist. Die Lifteinrichtung weist vorzugsweise einen Bewegungsmechanismus und/oder einen elektrisch ansteuerbaren Antriebsmechanismus zum Antreiben des Bewegungsmechanismus auf. Die Transporteinrichtung kann ferner einen beweglichen und elektrisch ansteuerbaren Greifarm zum Greifen und Halten mindestens eines Zellkulturbehälters sein. Die Transporteinrichtung kann ein Förderband zum Bewegen des mindestens einen darauf platzierten Zellkulturbehälters aufweisen. Durch den Transport kann der mindestens eine Zellkulturbehälter in der Inkubatorkammer bewegt werden, insbesondere zu einer Bearbeitungsposition in einer Bearbeitungsstation in der Inkubatorkammer, und von dieser Bearbeitungsposition weg. Die Steuereinrichtung kann dazu eingerichtet sein, die Transporteinrichtung in Abhängigkeit von Zellüberwachungsdaten zu steuern.

Die Bearbeitungsstation kann mindestens eine Messeinrichtung aufweisen, um den mindestens einen Wachstumsparameter zu messen, der das Wachstum der Zellen dieser Zellkultur charakterisiert. Wenn der mindestens eine Zellkulturbehälter mittels Transporteinrichtung im Inkubator bewegbar ist, können die Zellkulturen mehrerer Zellkulturbehälter mit einer einzigen oder mit wenigen Messeinrichtungen nacheinander gemessen werden. Es können aber auch mehrere oder eine Vielzahl von Messeinrichtungen vorgesehen sein, um parallel das Wachstum mehrerer Zellkulturen zu beobachten.

Die Messeinrichtung kann zudem an einer Transporteinrichtung befestigbar sein. Die Messeinrichtung kann an einem Positioniermechanismus befestigbar sein oder befestigt sein, mittels dem die Messeinrichtung in der Inkubatorkammer bewegbar und positionierbar ist. Der Positioniermechanismus kann einen beweglichen Roboterarm beinhalten und ist vorzugsweise elektrisch steuerbar, insbesondere durch ein Steuerprogramm der Steuereinrichtung. Auf diese Weise kann das Wachstum der Zellen mehrerer Zellkulturbehälter nacheinander mit einer oder mit wenigen Messeinrichtungen nacheinander gemessen werden. Der Positioniermechanismus kann als in die Inkubatorkammer einsetzbares Bauteil ausgebildet sein. Die Energiezufuhr dieses Bauteils kann über eine Kabelverbindung mit dem Inkubator erfolgen, vorzugsweise über ein durch eine Wandöffnung, z.B. einen Port, oder über eine solche Kabelverbindung zu einer externen Spannungsquelle. Die Steuereinrichtung kann dazu eingerichtet sein, den Positioniermechanismus in Abhängigkeit von Zellüberwachungsdaten zu steuern.

Als Behandlungseinrichtung lässt sich auch die Temperiereinrichtung der Inkubatorkammer verstehen, mit der die Atmosphäre im Inneren der Inkubatorkammer auf den gewünschten Wert, insbesondere 37°C geregelt wird. Der Begriff Temperieren bezieht sich auf das Erhöhen und Senken der Atmosphärentemperatur durch Heizen und Kühlen. Vorzugsweise wird die Temperatur im Inneren über eine Temperaturänderung der Wände des Inkubators eingestellt. Temperatursensoren der entsprechenden Temperaturregeleinrichtung sind an wenigstens einer Position im Inneren und / oder außerhalb der Inkubatorkammer, insbesondere an einer Wand der Inkubatorkammer verteilt.

Der Inkubator weist vorzugsweise eine Benutzerschnittstelleneinrichtung auf, über die der Benutzer Daten an die Datenverarbeitungseinrichtung bzw. die Steuereinrichtung eingeben kann, und/oder über die Informationen an den Benutzer ausgegeben werden können. Vorzugsweise ist der Inkubator bzw. diese Benutzerschnittstelleneinrichtung dazu eingerichtet, dass der Benutzer mindestens einen Betriebsparameter zum Betreiben der mindestens einen Messeinrichtung an dieser Benutzerschnittstelleneinrichtung eingeben kann bzw. Informationen von dieser erhalten kann. Auf diese Weise kann eine einzige Benutzerschnittstelleneinrichtung vom Benutzer dazu verwendet werden, um den Inkubator und auch die mindestens eine Messeinrichtung zu beeinflussen, oder zu steuern, oder von diesen Informationen zu erhalten. Insbesondere kann eine Messeinrichtung dazu eingerichtet sein, dem Benutzer auf eine mittels der Benutzerschnittstelleneinrichtung des Inkubators erfolgte Abfrage des Benutzers Zellüberwachungsdaten anzuzeigen, insbesondere eine Konfluenz, Zellanzahl, und/oder eine aus den Zellüberwachungsdaten abgeleitete statistische Information, und/oder mindestens ein optisches Abbild der mindestens einen Zellkultur anzuzeigen.

Eine gerätegesteuerte Behandlung des Inkubators ist vorzugsweise eine programmgesteuerte Behandlung, also eine durch ein Programm gesteuerte Behandlung. Unter einer programmgesteuerten Behandlung einer Probe ist zu verstehen, dass der Vorgang der Behandlung im Wesentlichen durch Abarbeiten einer Mehrzahl oder Vielzahl von Programmschritten erfolgt. Vorzugsweise erfolgt die programmgesteuerte Behandlung unter Verwendung mindestens eines Programmparameters, insbesondere mindestens eines vom Benutzer gewählten Programmparameters. Ein von einem Benutzer gewählter Parameter wird auch als Nutzerparameter bezeichnet. Die programmgesteuerte Behandlung erfolgt vorzugsweise mittels der digitalen Datenverarbeitungseinrichtung, die insbesondere Bestandteil der Steuereinrichtung ist. Die Datenverarbeitungseinrichtung kann mindestens einen Prozessor, d.h. eine CPU aufweisen, und/oder mindestens einen Mikroprozessor aufweisen. Die programmgesteuerte Behandlung wird vorzugsweise entsprechend den Vorgaben eines Programms gesteuert und/oder durchgeführt, insbesondere eines Steuerprogramms. Insbesondere ist bei einer programmgesteuerten Behandlung zumindest nach Erfassung der benutzerseitig erforderlichen Programmparameter im Wesentlichen keine Benutzertätigkeit erforderlich.

Unter einem Programmparameter wird eine Variable verstanden, die innerhalb eines Programms oder Unterprogramms, für mindestens eine Durchführung (Aufruf) des Programms oder Unterprogramms gültig, in vorbestimmter Weise eingestellt werden kann. Der Programmparameter wird, z.B. vom Benutzer, festgelegt und steuert das Programm oder Unterprogramm und bewirkt eine Datenausgabe in Abhängigkeit von diesem Programmparameter. Insbesondere beeinflusst und/oder steuert der Programmparameter und/oder steuern die vom Programm ausgegebenen Daten die Steuerung des Geräts, insbesondere die Steuerung der Behandlung mittels der mindestens einen Behandlungseinrichtung.

Unter einem Programm wird insbesondere ein Computerprogramm verstanden. Ein Programm ist eine Folge von Anweisungen, insbesondere bestehend aus Deklarationen und Instruktionen, um auf einer digitalen Datenverarbeitungseinrichtung eine bestimmte Funktionalität, Aufgaben- oder Problemstellung bearbeiten und/oder lösen zu können. Ein Programm liegt in der Regel als Software vor, die mit einer Datenverarbeitungseinrichtung verwendet wird. Das Programm kann insbesondere als Firmware vorliegen, im Fall der vorliegenden Erfindung insbesondere als Firmware der Steuereinrichtung des Inkubators oder des Systems. Das Programm liegt meist auf einem Datenträger als ausführbare Programmdatei, häufig im sogenannten Maschinencode vor, die zur Ausführung in den Arbeitsspeicher des Rechners der Datenverarbeitungseinrichtung geladen wird. Das Programm wird als Abfolge von Maschinen-, d. h. Prozessorbefehlen von dem/den Prozessoren des Computers verarbeitet und damit ausgeführt. Unter 'Computerprogramm' wird insbesondere auch der Quelltext des Programms verstanden, aus dem im Verlauf der Steuerung des Laborgerätes der ausführbare Code entstehen kann.

Eine Benutzerschnittstelleneinrichtung kann Bestandteil eines Inkubators sein, oder ein Modul. Eine Benutzerschnittstelleneinrichtung weist jeweils vorzugsweise auf: eine Steuereinrichtung für die Benutzerschnittstelleneinrichtung; eine Kommunikationseinrichtung zur Herstellung einer Datenverbindung mit einem Laborgerät, insbesondere einem Inkubator, über eine Schnittstelleneinrichtung desselben; eine Eingabeeinrichtung zur Erfassung von Benutzereingaben eines Benutzers; eine Ausgabeeinrichtung, insbesondere eine Anzeige und/oder ein Display, zur Ausgabe von Informationen an den Benutzer, insbesondere ein berührungsempfindliches Display. Dabei ist die Steuereinrichtung der Benutzerschnittstelleneinrichtung vorzugsweise dazu eingerichtet, über die Datenverbindung Daten mit der Steuereinrichtung des Inkubators auszutauschen.

Ein Zellkulturbehälter ist insbesondere aus Kunststoff, insbesondere PE oder PS gefertigt und weist insbesondere eine planare Bodenplatte auf, welche die Wachstumsfläche bildet. Diese kann eine Oberflächenbehandlung zur Förderung der Adhärenz von Zellen aufweisen. Der Zellkulturbehälter kann mit einer PE-Kappe oder Gasaustauschkappe, insbesondere einem Deckel mit optional enthaltenem Filter, verschließbar sein bzw. versehen sein. Der Zellkulturbehälter ist insbesondere stapelbar. Geeignet ist insbesondere eine Eppendorf Zellkulturflasche.

Die Erfindung bezieht sich insbesondere auf ein Datenaustauschsystem aufweisend mindestens einen erfindungsgemäßen Inkubator und mindestens eine externe Datenverarbeitungseinrichtung, wobei der mindestens eine Inkubator und die mindestens eine externe Datenverarbeitungseinrichtung für den Datenaustausch über eine Datenverbindung eingerichtet sind und insbesondere Zellüberwachungsdaten austauschen. Mit diesem Datenaustauschsystem lässt sich insbesondere die Datenerhebung wirkungsvoll gestalten, um Zellüberwachungsdaten mit einer Mehrzahl oder Vielzahl von erfindungsgemäßen Inkubatoren zu sammeln, insbesondere zentral mittels der externen Datenverarbeitungseinrichtung zu sammeln. Die externe Datenverarbeitungseinrichtung kann dazu eingerichtet sein, aus der Vielzahl gesammelter Zellüberwachungsdaten durch mindestens ein statistisches Verfahren Referenzdaten des Wachstums einer Zellart oder Zelllinie in Abhängigkeit von Zellüberwachungsdaten zu gewinnen, insbesondere um für bestimmte Wachstumsziele dieser Zellart oder Zelllinie die Abhängigkeiten von bestimmten ersten und zweiten Messwerten bzw. Zellüberwachungsdaten zu ermitteln.

Die Erfindung bezieht sich zudem auf ein System für das überwachte Wachstum von Zellkulturen, aufweisend: mindestens einen Inkubator, der zumindest aufweist: -eine Inkubatorkammer zur Aufnahme mindestens eines Zellkulturbehälters, der eine darin wachsende Zellkultur enthält, eine Sensoreinrichtung zur Messung mindestens eines Kammerparameters, der einen physikalischen Zustand der Inkubatorkammer charakterisiert, in Form mindestens eines ersten Messwertes, mindestens eine Messeinrichtung zur Messung mindestens eines Wachstumsparameters, der das Wachstum der Zellen dieser Zellkultur charakterisiert, in Form mindestens eines zweiten Messwertes, wobei das System weiter aufweist: -eine Datenspeichereinrichtung, und - eine Datenverarbeitungseinrichtung, die zum Erfassen des mindestens einen ersten Messwertes und des mindestens einen zweiten Messwertes in Form von Zellüberwachungsdaten und zum Speichern der Zellüberwachungsdaten in der Datenspeichereinrichtung eingerichtet ist. Dies bedeutet, dass die Erfassung und Zusammenführung der ersten und zweiten Messwerte hier nicht zwingend im Inkubator erfolgt, sondern in der externen Datenverarbeitungseinrichtung des Systems, die nicht Bestandteil des Inkubators ist. Auf diese Weise können insbesondere durch mehrere in einem Labor- oder Unternehmens-Laborgerätenetzwerk datenverbundenen Inkubatoren die Zellüberwachungsdaten zentral von der externen Datenverarbeitungseinrichtung gesammelt und gespeichert werden.

Die Erfindung bezieht sich zudem auf ein Verfahren für das Überwachen des Wachstums mindestens einer Zellkultur in einem Inkubator, aufweisend die Schritte: Messen mindestens eines Kammerparameters der Inkubatorkammer des Inkubators, wobei der Kammerparameter einen physikalischen Zustand der Inkubatorkammer charakterisiert, in Form mindestens eines ersten Messwertes, mittels mindestens einer Sensoreinrichtung des Inkubators, Messen mindestens eines Wachstumsparameters, der das Wachstum der Zellen dieser Zellkultur charakterisiert, in Form mindestens eines zweiten Messwertes, mittels mindestens einer Messeinrichtung des Inkubators, Erfassen des mindestens einen ersten Messwertes und des mindestens einen zweiten Messwertes in Form von Zellüberwachungsdaten mittels einer Datenverarbeitungseinrichtung des Inkubators oder eines Systems und Speichern der Zellüberwachungsdaten mittels einer Datenspeichereinrichtung des Inkubators oder eines Systems, wobei das System diesen Inkubator aufweist. Ein weiterer optionaler Schritt des Verfahrens besteht darin, mindestens einen Arbeitsschritt des Inkubators in Abhängigkeit von Zellüberwachungsdaten auszuführen. Das erfindungsgemäße Verfahren ist vorzugsweise vom erfindungsgemäßen Inkubator oder System ausführbar. Das erfindungsgemäße Verfahren liegt vorzugsweise als Programmcode vor, der die genannten Schritte bewirkt, wenn der Programmcode auf der Datenverarbeitungseinrichtung des erfindungsgemäßen Inkubators oder Systems ausgeführt wird. Der erfindungsgemäße Inkubator oder das System sind vorzugsweise dazu eingerichtet, das erfindungsgemäße Verfahren oder den Programmcode auszuführen.

Weitere bevorzugte Ausgestaltungen des erfindungsgemäßen Verfahrens lassen sich aus der Beschreibung des erfindungsgemäßen Inkubators und dessen bevorzugte Ausgestaltungen entnehmen. Ferner ergeben sich weitere Ausgestaltungsoptionen der Erfindung aus den Ausführungsbeispielen in den Figuren.

Es zeigen:
Fig. 1a zeigt eine perspektivische Frontansicht eines erfindungsgemäßen Inkubators gemäß Ausführungsbeispiel.
Fig. 1b zeigt eine perspektivische Rückansicht des Inkubators der Fig. 1a.
Fig. 2 zeigt eine schematische Ansicht eines erfindungsgemäßen Inkubators gemäß einem weiteren Ausführungsbeispiel.
Fig. 3 zeigt schematisch das erfindungsgemäße Verfahren gemäß Ausführungsbeispiel.

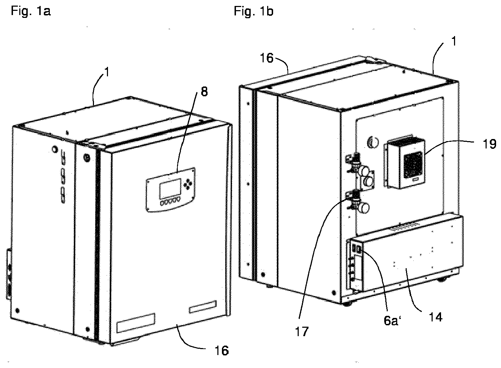

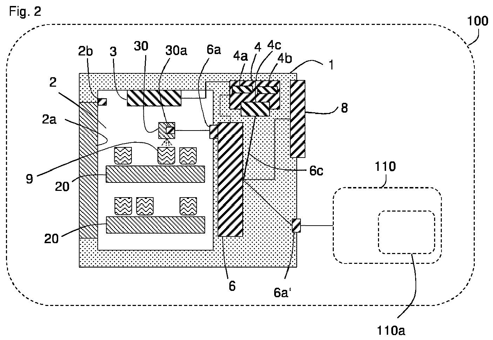

Fig. 1a zeigt den Inkubator 1 für das überwachte Wachstum von Zellkulturen, hier ein CO2-Inkubator für das Wachstum von eukaryotischen Zellen. Der Inkubator besitzt: eine Inkubatorkammer 2 (siehe Figur 2) zur Aufnahme mindestens eines Zellkulturbehälters 9, der eine darin wachsende Zellkultur enthält, eine Sensoreinrichtung 3 zur Messung der Kammerparameter Temperatur, relative CO2-Gaskonzentration und relative Luftfeuchtigkeit. Die Werte dieser Kammerparameter werden durch das Steuerprogramm der Steuereinrichtung 4 des Inkubators als erste Messwerte regelmäßig eingelesen. Der Inkubator 1 weist zudem eine Messeinrichtung 30 zur Messung des Wachstumsparameters "Konfluenz des Zellrasens im Zellkulturbehälter" als zweite Messwerte auf. Dies erfolgt durch eine Beleuchtung und Detektion des Zellrasens, bzw. optional eine Abbildung des Zellrasens, veranlasst durch die Steuereinrichtung 4 in regelmäßigen Zeitabständen. Die Bilder des Zellrasens werden von der Datenverarbeitungseinrichtung der Steuereinrichtung 4 durch softwaregestützte Bildanalyseverfahren ausgewertet und die Zelldichte wird ermittelt.

Die Messeinrichtung 30 ist hier ein Netzwerkinstrument, das über einen Stecker 6a des Ethernet-Switch 6 des Inkubators in Datenverbindung 6c mit der Steuereinrichtung bzw. deren Ethernet-Adapter 4c steht. Die Zellkulturbehälter 9 sind auf in der der Inkubatorkammer befestigten Regalplatten 20 angeordnet. Die Inkubatorkammer 2 ist über eine Inkubatortüre 2a zugänglich. Das Öffnen der Inkubatortüre kann über einen Sensor 2b von der Steuereinrichtung erfasst werden.

Die Steuereinrichtung 4 weist eine Datenspeichereinrichtung 4a, und eine Datenverarbeitungseinrichtung 4b auf, die zum Erfassen des mindestens einen ersten Messwertes und des mindestens einen zweiten Messwertes in Form von Zellüberwachungsdaten und zum Speichern der Zellüberwachungsdaten in der Datenspeichereinrichtung eingerichtet sind.

Der Inkubator 1 sammelt so die Informationen über das Wachstum der Zellen in der Inkubatorkammer 2 und gewinnt auf diese Weise Referenzdaten, die eine standardisierte Vorgehensweise für die Zellkultur in dem Inkubator erlauben. Die hochwertige und präzise kalibrierte Sensorik 3 des Inkubators bietet die Grundlage zur Erhebung eines verlässlichen Datensatzes zur Charakterisierung des Zellwachstums in diesem Inkubator. Es werden dabei die inkubator-internen Parameter, insbesondere Kammerparameter, gemeinsam mit den durch die Messeinrichtung gemessenen Wachstumsparametern gesammelt, so dass eine Ermittlung von eventuellen Korrelationen zwischen Parameterwerten und deren Änderungen ermöglicht wird. Auf diese Weise werden im Inkubator Zellüberwachungsdaten gewonnen, die als Referenzdaten zur Einschätzung der Entwicklung zukünftig im Inkubator zu wachsender Zellkulturen einsetzbar sind.

Anwender arbeiten in der Zellkultur mit eukaryotischen Zellen in CO2-Inkubatoren. Reproduzierbares Verhalten dieser Zellen in Testsystemen wird im Stand der Technik oftmals durch subjektive Eindrücke durch morphologische Überprüfung oder Endpunktbestimmungen (Zellzählung) unterstützt. Zusätzlich erfolgt keine automatische Dokumentation der Ergebnisse der Zellen im Inkubator in Verbindung mit den Umgebungsbedingungen, die der Inkubator 1 direkt zur Verfügung stellt. Das klassische Mikroskopieren der Kulturen deckt keine vollständige Überwachung der Zellen ab, sondern bildet nur Momentaufnahmen ab. Zusätzlich bieten sie zumeist keine quantifizierbaren Aussagen. Dieses Zellzählen des Stands der Technik ist ein disruptiver Prozess, der nicht während des Wachstums erfolgt.

Der Inkubator 1 ist hier zudem dazu eingerichtet, mittels des Steuerprogramms der Steuereinrichtung 4 ein Planungsprogramm auszuführen, das aus Zellüberwachungsdaten und bekannten Referenzdaten den zeitabhängigen Verlauf des Wachstums (Konfluenz) vorhersagt und dem Benutzer über die Benutzerschnittstelleneinrichtung 8 die Information über das Erreichen des gewünschten Wachstumsziels im Display anzeigt, hier eine vom Benutzer vorgegebene Konfluenz.

Die Zellüberwachungsdaten werden zudem optional vom Inkubator bzw. dessen Steuereinrichtung über eine Ethernet-Schnittstelle 6a' an eine externe Datenverarbeitungseinrichtung 110 übertragen, die auch gegebenenfalls von weiteren Inkubatoren (nicht gezeigt) desselben Netzwerks 100 Zellüberwachungsdaten sammelt und speichert. Dadurch können Zellüberwachungsdaten im externen Gerät zentral zusammengeführt werden und statistisch ausgewertet werden, um Referenzdaten für das Normwachstum einer bestimmten Zellart oder Zelllinie zu gewinnen.

In einer alternativen Ausführungsform der Erfindung erfolgt die Erfassung der ersten zusammen mit den zweiten Messwerten bzw. deren Zusammenführung in Form von Zellüberwachungsdaten nicht in der Datenverarbeitungseinrichtung 4b des Inkubators, sondern in einer zweiten externen Datenverarbeitungseinrichtung 110. Auf diese Weise kann die Messeinrichtung 30, insbesondere unabhängig vom Inkubator 1 oder dessen Steuereinrichtung 4, die zweiten Messwerte an die externe Datenverarbeitungseinrichtung 110 übertragen, insbesondere mit einem Zeitstempel der Messung, und die Steuereinrichtung 4 kann die mittels Sensoreinrichtung 3 gemessene erste Messwerte, insbesondere mit einem Zeitstempel der Messung, an die externe Datenverarbeitungseinrichtung 110 übertragen. Diese bildet aus den ersten und zweiten Messwerten die Zellüberwachungsdaten und speichert diese. Die gespeicherten Zellüberwachungsdaten oder daraus von der Datenverarbeitungseinrichtung 110 gewonnene Referenzdaten werden den Netzwerkinstrumenten des Netzwerks, insbesondere den Inkubatoren, wieder bereitgestellt. Auf diese Weise wird ein Datenaustauschsystem bzw. ein erfindungsgemäßes System bereitgestellt, mit dem sich das Erreichen von Wachstumszielen standardisiert erreichen lässt.

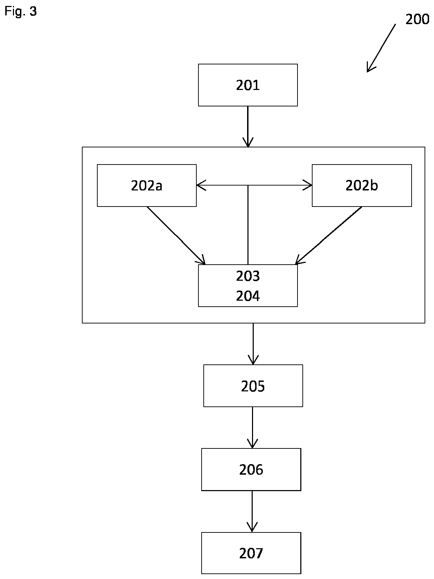

Das in Fig. 3 gezeigte Verfahren 200 für das Überwachen des Wachstums mindestens einer Zellkultur in einem Inkubator umfasst die Schritte:
Verfahren für das Überwachen des Wachstums mindestens einer Zellkultur in einem Inkubator, aufweisend die Schritte:
   Erfassen eines vom Benutzer über eine Benutzerschnittstelleneinrichtung eingegebenen Wachstumsziels, z.B. einer gewünschten Konfluenz, insbesondere bei einer vom Benutzer eindeutig vorgegebenen Zellinie (201).

Messen mindestens eines Kammerparameters der Inkubatorkammer des Inkubators, wobei der Kammerparameter einen physikalischen Zustand der Inkubatorkammer charakterisiert, in Form mindestens eines ersten Messwertes, mittels mindestens einer Sensoreinrichtung des Inkubators (202a).
Messen mindestens eines Wachstumsparameters, der das Wachstum der Zellen dieser Zellkultur charakterisiert, in Form mindestens eines zweiten Messwertes, mittels mindestens einer Messeinrichtung des Inkubators (202b).

Erfassen des mindestens einen ersten Messwertes und des mindestens einen zweiten Messwertes in Form von Zellüberwachungsdaten mittels einer Datenverarbeitungseinrichtung (203) und Auswerten der Zellüberwachungsdaten (204), hier durch Vergleich mit bekannten Referenzdaten.

Wiederholen der Schritte 202a, 202b, 203, 204, bis das Wachstumsziel erreicht ist bzw. bis das Zellwachstum vom Anwender beendet wird.

Speichern der Zellüberwachungsdaten mittels einer Datenspeichereinrichtung (205).

Festlegen von neuen Referenzdaten anhand der Zellüberwachungsdaten (206).

Bereitstellen der neuen Referenzdaten für den Inkubator für die zukünftige Inkubation (207).

Das Überwachen von Zellen im Inkubator 1, und das Berücksichtigen von Bewegen und Stören der Zellkulturen während des Wachstums, ist Teil der Funktionen des Inkubators 1 und kann von diesem gesteuert werden. Außerdem werden automatisch die Umgebungsbedingungen (erste Messwerte) an die Zellergebnisse (zweite Messwerte) verknüpft und optional dokumentiert. Somit ist es möglich, schnell und einfach Wachstumsverhalten von Zellen zu standardisieren.

Ein CO2-Inkubator ist mit der Messstation 3 ausgerüstet, die Informationen über das Wachstum von Zellen (Konfluenz und optional Zellzahl) automatisch aufnimmt. Diese Information wird direkt im Inkubator 1 oder vom System gespeichert und liegt somit zusätzlich zu den Informationen der Umgebungsbedingungen (erste Messwerte) im Inkubator vor. Somit sind die Informationen des Inkubators direkt mit den Zelldaten (zweite Messwerte) verknüpft.

Weitere Methoden sind vorteilhaft, um die Standardisierung der Zellkultur mit einem solchen Inkubator bzw. System zu optimieren:
Nach dem Auftauprozess von Zellen kann der Anwender die Zeit bestimmen lassen, wann alle oder ein definierter Anteil der Zellen auf der Oberfläche eines Zellkulturgefäßes adhäriert sind. Der Benutzer bekommt zu diesem Zeitpunkt eine Information. Deren Abweichung von bekannten Normwerten wird dem Benutzer vorzugsweise signalisiert oder als Daten gespeichert.
Der Anwender bekommt eine Information über die Menge der Zellen, die nach Aussaat oder Auftauprozess angeheftet vorliegen. Bei eingegebener Gesamtzellzahl könnte auch die Überlebensrate ermittelt werden. Der Anwender bekommt eine Mitteilung, wann seine gewünschte Konfluenz der Zellen erreicht wird, um beispielsweise die Zellen zu manipulieren und kann diesen Wert mit den bereits abgespeicherten Werten vergleichen, um eine Standardisierung zu erzielen.
Der Anwender kann ab einer zu definierenden Anzahl von gemessenen Proben automatisch Bandbreiten bzw. Referenzbereiche ermitteln lassen, in denen die typischen Messwerte einer Zelllinie liegen müssen.
Der Anwender kann den Inkubator nutzen, um das Wachstum von Zellen zu überwachen. Er kann die Messeinrichtung 30 direkt an der Bedieneinheit 8 des Inkubators steuern und die Ergebnisse des Zellwachstums und die Umgebungsbedingungen sowie das Eventlog des Inkubators verknüpft dokumentieren - dies erfolgt vorzugsweise automatisch. Indem die Ergebnisse direkt im Inkubator gespeichert vorliegen, können Wachstumskurven standardisiert aufgenommen werden. Wenn der Anwender seinen Zelltyp spezifiziert, wird die aktuelle Wachstumskurve direkt mit historischen Daten verglichen und dokumentiert. Die Abweichung kann der Anwender automatisch durch einen Auswertungsalgorithmus als gut oder schlecht bewerten lassen. Bei Abweichungen von erwarteten Ergebnissen, kann der Anwender direkt handeln und dies später dokumentieren. Dadurch, dass die Messeinrichtung 30 Teil des Inkubators ist, ist eine ein-eindeutige Aufzeichnung der Umgebungsbedingungen der Zellkultur gewährleistet.
Zusätzlich kommt hinzu, dass der Inkubator 1 optional in ein Netzwerk 100 integriert ist und die ersten und zweiten Messwerte an eine Datenbank einer externen Datenverarbeitungseinrichtung sendet. Wenn der Inkubator bzw. das System diese Zellüberwachungsdaten speichert, stehen sie allen Anwendern des Netzwerks zur Verfügung. Wenn die Zellüberwachungsdaten z.B. in einer Cloud gespeichert werden, können sie allen Anwendern des globalen Netzwerks zugänglich gemacht werden. Somit können kombinierte Zell- und Umgebungsbedingungsdaten von Arbeitsgruppe zu Arbeitsgruppe über die gesamte Welt automatisch transferiert werden. Dies ist vorteilhaft insbesondere deshalb, da die Kooperation zwischen Anwendern im wissenschaftlichen Sektor sehr wichtig ist. Je valider die Zellüberwachungsdaten bzw. Referenzdaten werden und die Rückführbarkeit der Ergebnisse zunimmt, desto einfacher und schneller führt die Zusammenarbeit in diesem Bereich zu zuverlässigen Ergebnissen. Die Zellkultur ist bekannt dafür, dass Standardisierung von Methoden und Reproduzierbarkeit von Ergebnissen essentiell und momentan nicht optimal sind. Eine globale Datenbank von kombinierten Zelldaten und Umgebungsbedingungsdaten, die erfindungsgemäß als Zellüberwachungsdaten zuverlässig ermittelt werden, weil sie auf zuverlässigen Inkubatordaten basieren, ermöglicht einen effizienten Transfer von Protokollen und Ergebnissen.

## Patentansprüche

1. Inkubator (1) für das überwachte Wachstum von Zellkulturen, aufweisend
eine Inkubatorkammer (2) zur Aufnahme mindestens eines Zellkulturbehälters (9), der eine darin wachsende Zellkultur enthält,
eine Sensoreinrichtung (2b; 3) zur Messung mindestens eines Kammerparameters, der einen physikalischen Zustand der Inkubatorkammer charakterisiert, in Form mindestens eines ersten Messwertes,
mindestens eine Messeinrichtung (30) zur Messung mindestens eines Wachstumsparameters, der das Wachstum der Zellen dieser Zellkultur charakterisiert, in Form mindestens eines zweiten Messwertes,
eine Datenspeichereinrichtung (4a), und eine Datenverarbeitungseinrichtung (4b), die zum Erfassen des mindestens einen ersten Messwertes und des mindestens einen zweiten Messwertes in Form von Zellüberwachungsdaten und zum Speichern der Zellüberwachungsdaten in der Datenspeichereinrichtung eingerichtet ist.

2. Inkubator gemäß Anspruch 1, wobei die Sensoreinrichtung (2b) dazu eingerichtet ist, das Öffnen und/oder Schließen der Inkubatortüre (2a) als Kammerparameter in Form des mindestens einen ersten Messwerts zu erfassen.

3. Inkubator gemäß Anspruch 1 oder 2, wobei die Datenverarbeitungseinrichtung (4b) des Inkubators die Durchführung der Messung des mindestens einen zweiten Messwertes steuert.

4. Inkubator gemäß einem der vorangehenden Ansprüche, wobei der Wachstumsparameter aus der Gruppe der bevorzugten Wachstumsparameter entnommen ist, umfassend:
- Konfluenz des Zellrasens im Zellkulturbehälter,
- Anzahl der Zellen in einem Testabschnitt des Zellkulturbehälters,
- Morphologie der Zellen des Zellrasens,
- Zelladhäsion.

5. Inkubator gemäß einem der vorangehenden Ansprüche, wobei der Inkubator und/oder dessen Datenverarbeitungseirichtung (4b) und/oder dessen eine Steuereinrichtung (4) und/oder die mindestens eine Messeinrichtung (30) dazu eingerichtet sind, die Zellüberwachungsdaten gemäß mindestens einer der nachstehend genannten Konfigurationen zu erfassen:
- Es werden Zellüberwachungsdaten zu Zeitpunkten gemäß einem vordefinierten Zeitplan erfasst, der vom Inkubator festgelegt worden sein kann und/oder vom Benutzer über eine Benutzerschnittstelleneinrichtung des Inkubators beeinflusst oder festgelegt worden sein kann;
- Es werden Zellüberwachungsdaten in vorgegebenen Zeitabständen, insbesondere regelmäßigen Zeitabständen erfasst, die vom Inkubator festgelegt worden sein können und/oder vom Benutzer über eine Benutzerschnittstelleneinrichtung des Inkubators beeinflusst oder festgelegt worden sein können.

6. Inkubator gemäß einem der vorangehenden Ansprüche, wobei der Inkubator und/oder dessen Datenverarbeitungseinrichtung (4b) und/oder dessen Steuereinrichtung (4) und/oder die mindestens eine Messeinrichtung (30) dazu eingerichtet sind, dass die Zellüberwachungsdaten in Abhängigkeit von mindestens einem vorgegebenen Ereignis erfasst werden, das aus der Gruppe der folgenden Ereignisse ausgewählt ist:
∘ ein mittels der Sensoreinrichtung gemessener erster Messwert erfüllt eine vorgegebene Bedingung, z.B. eine unerlaubte Abweichung von der Solltemperatur in der Inkubatorkammer, die z.B. durch eine geöffnete Inkubatortüre verursacht sein kann, oder eine unerlaubte Abweichung von einer relativen Gaskonzentration in der Inkubatorkammer, insbesondere ein CO₂ und/oder O₂-Wert; oder eine vorgebene Anzahl an Türöffnungen (innerhalb eines vorgegebenen Zeitraums); oder eine unerlaubte Abweichung von einer Luftfeuchtigkeit;
∘ ein vom Inkubator erkannter Fehlerzustand des Inkubators, betreffend Hardware oder Software;
∘ Es wird über eine Kommunikationseinrichtung der Steuereinrichtung des Inkubators oder der Messeinrichtung ein Steuersignal empfangen, das die Erfassung von Zellüberwachungsdaten auslöst; ein solches Steuersignal kann insbesondere von einer externen Datenverarbeitungseinrichtung veranlasst sein oder durch eine Benutzereingabe veranlasst worden sein.

7. Inkubator gemäß einem der vorangehenden Ansprüche, wobei der Inkubator und/oder dessen Datenverarbeitungseirichtung (4b) und/oder dessen Steuereinrichtung (4) und/oder die mindestens eine Messeinrichtung (30) dazu eingerichtet sind, dass ein von der Datenverarbeitungseinrichtung oder der Steuereinrichtung des Inkubators oder der Messeinrichtung ausgeführtes Steuerprogramm eine Messung von Zellüberwachungsdaten auslöst, nachdem ein vom Steuerprogramm angewandtes Auswertungsverfahren zuvor gemessene zweite Messwerte ausgewertet hat.

8. Inkubator gemäß Anspruch 7, wobei ein Auswertungsprogramm die Auslösung der Messung von Zellüberwachungsdaten aufgrund einer erfüllten Bedingung vorsehen, diese Bedingung kann aus der Gruppe der folgenden Bedingungen ausgewählt sein:
∘ Mindestens ein zuvor gemessener zweiter Messwert weicht von mindestens einem Referenzwert ab oder liegt außerhalb eines vordefinierten Bereichs;
∘ Mindestens ein Verlauf aus zwei oder vielen zuvor gemessenen zweiten Messwerten weicht von mindestens einem Referenzverlauf ab oder liegt außerhalb eines vordefinierten Referenzbereichs; dieser Referenzverlauf oder Referenzbereich kann ein bekannter Normverlauf oder Normbereich sein, der insbesondere von anderen Parametern abhängen kann, insbesondere der Information über die Zellart und/oder das für die betreffende Zellkultur verwendete Zellmedium.

9. Inkubator gemäß einem der vorangehenden Ansprüche, wobei der Inkubator eine Benutzerschnittstelleneinrichtung (8) aufweist, über die sowohl der Inkubator als auch die mindestens eine Messeinrichtung vom Benutzer steuerbar ist.

10. Datenaustauschsystem aufweisend
mindestens einen Inkubator (1) gemäß Anspruch 1 und
mindestens eine externe Datenverarbeitungseinrichtung (110),
wobei der mindestens eine Inkubator und die mindestens eine externe Datenverarbeitungseinrichtung für den Datenaustausch über eine Datenverbindung eingerichtet sind und insbesondere Zellüberwachungsdaten austauschen.

11. System (100) für das überwachte Wachstum von Zellkulturen, aufweisend:
mindestens einen Inkubator, der zumindest aufweist:
- eine Inkubatorkammer zur Aufnahme mindestens eines Zellkulturbehälters, der eine darin wachsende Zellkultur enthält,
- eine Sensoreinrichtung zur Messung mindestens eines Kammerparameters, der einen physikalischen Zustand der Inkubatorkammer charakterisiert, in Form mindestens eines ersten Messwertes,
- mindestens eine Messeinrichtung zur Messung mindestens eines Wachstumsparameters, der das Wachstum der Zellen dieser Zellkultur charakterisiert, in Form mindestens eines zweiten Messwertes,
wobei das System weiter aufweist:
- eine Datenspeichereinrichtung (110), und
- eine Datenverarbeitungseinrichtung (110a), die zum Erfassen des mindestens einen ersten Messwertes und des mindestens einen zweiten Messwertes in Form von Zellüberwachungsdaten und zum Speichern der Zellüberwachungsdaten in der Datenspeichereinrichtung eingerichtet ist.

12. Verfahren (200) für das Überwachen des Wachstums mindestens einer Zellkultur in einem Inkubator, aufweisend die Schritte:
Messen mindestens eines Kammerparameters der Inkubatorkammer des Inkubators, wobei der Kammerparameter einen physikalischen Zustand der Inkubatorkammer charakterisiert, in Form mindestens eines ersten Messwertes, mittels mindestens einer Sensoreinrichtung des Inkubators, (202a)
Messen mindestens eines Wachstumsparameters, der das Wachstum der Zellen dieser Zellkultur charakterisiert, in Form mindestens eines zweiten Messwertes, mittels mindestens einer Messeinrichtung des Inkubators, (202b)
Erfassen des mindestens einen ersten Messwertes und des mindestens einen zweiten Messwertes in Form von Zellüberwachungsdaten mittels einer Datenverarbeitungseinrichtung (203) und
Speichern (205) der Zellüberwachungsdaten mittels einer Datenspeichereinrichtung.

13. Verfahren gemäß Anspruch 12, das zusätzlich den Schritt aufweist:
- Ausführen eines Arbeitsschritts des Inkubators in Abhängigkeit von den Zellüberwachungsdaten.

14. Verfahren gemäß Anspruch 12 oder 13, das zusätzlich den Schritt aufweist:
- Ermitteln einer Korrelation zwischen Zellüberwachungsdaten, insbesondere ersten und zweiten Messwerten und Speichern der Information über diese Korrelation.

15. Verfahren gemäß Anspruch 13, wobei der Arbeitsschritt vorsieht, dass ein Planungsprogramm zur Berechnung eines Wachstumsziels von einer Datenverarbeitungseinrichtung ausgeführt wird.

16. Verfahren gemäß Anspruch 13, wobei der Arbeitsschritt vorsieht, dass dem Benutzer an einer Benutzerschnittstelleneinrichtung (8) der von einer Datenverarbeitungseinrichtung (4b; 110a) in Abhängigkeit von den Zellüberwachungsdaten berechnete Zeitpunkt des Erreichens eines Wachstumsziels angezeigt oder anders ausgegeben wird.
